# EUROPEAN PATENT SPECIFICATION

(11) **EP 3 082 799 B1**
(45) Date of publication and mention of the grant of the patent: **28.04.2021**
(21) Application number: 14871042.9
(22) Date of filing: 17.12.2014
(51) Int. Cl.: A61K 31/352, A61P 31/12, A61P 31/18, A61P 31/14, A61K 31/7048, A61K 31/427, A61K 31/513, A61K 31/4418, A61K 31/635, A61K 31/675, A61K 31/7068, A61K 38/19, A61K 38/20, A61K 31/536, A61K 45/06

(54) **USE OF ISOFLAVONES FOR THE TREATMENT OF RETROVIRAL INFECTION**
VERWENDUNG VON ISOFLAVONEN ZUR BEHANDLUNG RETROVIRALER INFEKTION
UTILISATION D'ISOFLAVONES POUR LE TRAITEMENT D'INFECTION RÉTROVIRALE

(30) Priority: 17.12.2013 US 201361917175 P
(43) Date of publication of application: 26.10.2016
(73) Proprietor: Versitech Limited, Hong Kong (CN)
(72) Inventor: LIU, Li, Hong Kong (CN); CHEN, Zhiwei, Hong Kong (CN); CHEN, Jianping, Hong Kong (CN); LIANG, Jianguo, Hong Kong (CN); ZHENG, Xiao, Hong Kong (CN); WANG, Dongmei, Guangzhou Guangdong (CN); YANG, De Po, Guangzhou Guangdong (CN)
(74) Representative: HGF
(86) International application number: PCT/CN2014/094083
(87) International publication number: WO 2015/090201

(56) References cited:
- WO-A1-2012/035321
- CN-A- 101 279 993
- CN-A- 102 302 484
- JP-A- 2001 253 823
- Anonymous: "Management of HIV/AIDS - Wikipedia, the free encyclopedia", , 24 October 2013 (2013-10-24), XP055385627, Retrieved from the Internet: URL:https://web.archive.org/web/2013102409 5335/https://en.wikipedia.org/wiki/Managem ent_of_HIV/AIDS [retrieved on 2017-06-27]
- BRODER ET AL: "The development of antiretroviral therapy and its impact on the HIV-1/AIDS pandemic", ANTIVIRAL RESEARCH, ELSEVIER BV, NL, vol. 85, no. 1, 1 January 2010 (2010-01-01), pages 1-18, XP026835799, ISSN: 0166-3542 [retrieved on 2009-12-16]
- ANDRES A ET AL: "Soy isoflavones and virus infections", THE JOURNAL OF NUTRITIONAL BIOCHEMISTRY, ELSEVIER, AMSTERDAM, NL, vol. 20, no. 8, 1 August 2009 (2009-08-01) , pages 563-569, XP026283511, ISSN: 0955-2863, DOI: 10.1016/J.JNUTBIO.2009.04.004 [retrieved on 2009-07-09]
- HIDAYAT HUSSAIN ET AL: "A patent review of the therapeutic potential of isoflavones (2012-2016)", EXPERT OPINION ON THERAPEUTIC PATENTS., 13 June 2017 (2017-06-13), pages 1-12, XP055383074, GB ISSN: 1354-3776, DOI: 10.1080/13543776.2017.1339791

## Description

### FIELD OF THE INVENTION

The present invention is associated in part with a method of treating a retroviral infection, for example, HIV infection, by administering an agent, for example, isoflavones.

### BACKGROUND OF THE INVENTION

A latent virus is a non-active virus present within a cell. Many Retroviruses have the capacity to be present as latent viruses in a host's cells. Latent viruses do not produce rapid lysis of host cells, but rather remain latent for long periods in the host before clinical symptoms appear. The term latency is used to denote the interval from infection to clinical manifestations. Upon initiation by a specific trigger, the latent viruses can begin replicating and cause diseases. Many antiviral drugs cannot eliminate latent viruses; however, are effective against actively replicating viruses.

Human immunodeficiency virus (HIV), a lentivirus (slowly replicating retrovirus), is an example of a virus capable of being present in a host over a long time in a latent stage. HIV is the pathogenic agent of acquired immunodeficiency syndrome (AIDS). AIDS causes a progressive failure of the human immune system triggering the opportunistic infections. Currently, AIDS is one of the most devastating human diseases worldwide (Barresinoussi et al., 1983; UNAIDS, 2012). By the end of 2011, approximately 34 million people lived with HIV, 2.5 million were newly infected within the year and 1.7 million died of AIDS (UNAIDS, 2012).

Although "highly active anti-retroviral therapy" (HAART) exhibits ability to suppress HIV-1 level to undetectable level, this therapy does not efficiently reduce the residual, latent pro-viral DNA integrated into the host cellular genome (Chun et al., 1997; Finzi et al., 1997; Wong et al., 1997). HIV-1 is able to establish a latently infected status at the level of T cells (R. F. Siliciano & Greene, 2011). When activated CD4⁺ T cells are infected by HIV-1 and become a long-lived resting memory state, no viral replication is permissive. Extremely slow pace of decay of HIV-1 reservoir is observed leading to treat HIV-1 infected individuals over 60 years (Finzi et al., 1999; J. D. Siliciano et al., 2003; Strain et al., 2003). Therefore, HAART alone cannot eliminate HIV-1. HIV-1 viremia rebounds when the antiretroviral therapy is interrupted. HIV-1 reservoir creates a barrier for HIV-1 eradication making HAART impractical.

The molecular mechanisms of latency are complex and include the absence in resting CD4⁺ T cells of nuclear forms of key host transcription factors (e.g., NFkB, Sp1, and NFAT), the absence of Tat and associated host factors that promote efficient transcriptional elongation, epigenetic changes inhibiting HIV-1 gene expression, and transcriptional interference (Graci, Colacino, Peltz, Dougherty, & Gu, 2009; R. F. Siliciano & Greene, 2011). Molecular studies had established that transcription from the HIV-1 LTR was stimulated by inducible host transcription factors (Contreras et al., 2009; Folks et al., 1986; Graci et al., 2009; R. F. Siliciano & Greene, 2011). The clinical attempts for purging latently infected cells have been made using immune activation tactics. Small molecules are also investigated to reactivate HIV-1 from latency.

WO 2012/035321 A1 discloses an anti-microtubule agent for use in a method of treating a subject having a latent virus, the method comprising: administering the anti-microtubule agent and an anti-viral agent to the subject.

JP 2001 253823 A discloses a HIV promoter activity inhibitor containing a compound having an isoflavone skeleton, a 3-phenylcoumarin skeleton or a chalcone skeleton, or their glycosides.

Novel intervention methods that activate latent viruses, for example latent retroviruses such as HIV, are desirable. In HIV infected subjects, activation of HIV-1 expression in the combination with HAART is expected to result in T-cell apoptosis or disruption of a cell expressing viral proteins by the host immune system, and thus depletion of the persistent HIV-1 reservoir. Such interventions would not just inhibit but can also eliminate HIV infection.

### BRIEF SUMMARY OF THE INVENTION

The present invention relates to pharmaceutical preparations and methods for reactivating latent retroviral infections, for example, HIV infection. The present invention is based, in part, on the discovery that isoflavones and derivatives thereof, for example Daidzein (DDZ), can be used to activate the expression of quiescent, integrated retroviruses, for example, HIV, within latently infected cells, thereby depleting latent retroviral infection. Further, selective induction of latent retroviral infection would allow other antiretroviral drugs and the antiviral immune responses to access and eliminate residual retroviral infection.

In accordance with an aspect of the invention there is provided a compound selected from the group consisting of: 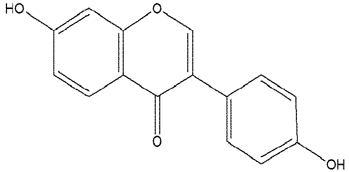 4',7-Dihydroxyisoflavone (Daidzein), 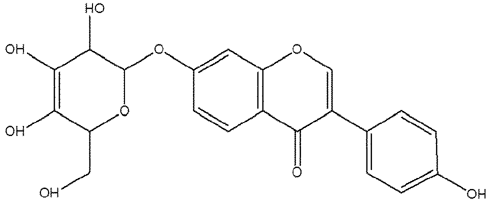 Daidzein 7-glucoside (Daidzin), 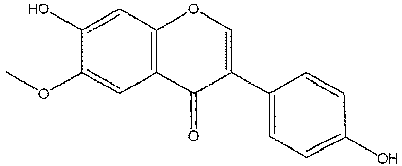 4',7-Dihydroxy-6-methoxyisoflavone (Glycitein),

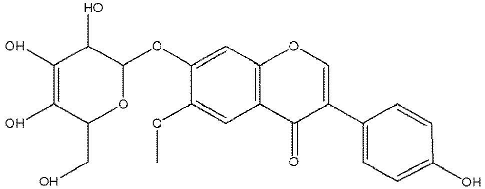

Glycitein 7-glucoside (Glycitin), or combinations thereof for use in reactivating a latent retroviral infection in a subject.

In accordance with a further aspect of the invention there is provided a compound selected from the group consisting of: 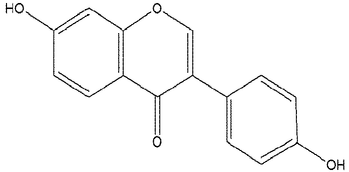 4',7-Dihydroxyisoflavone (Daidzein), 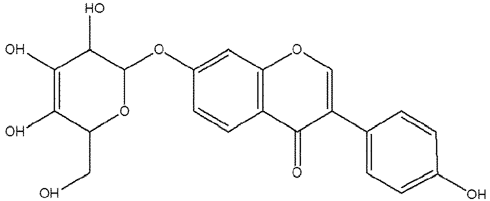 Daidzein 7-glucoside (Daidzin), 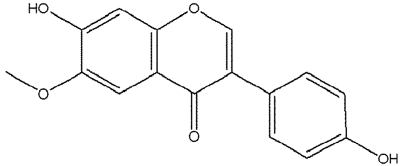 4',7-Dihydroxy-6-methoxyisoflavone (Glycitein),

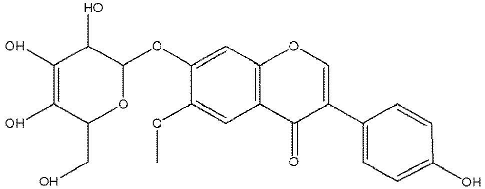

Glycitein 7-glucoside (Glycitin), or combinations thereof,
for use in treating a retroviral infection in a subject, the treatment further comprising administering a therapy directed to stimulating the immune system of the subject.

In accordance with another aspect of the invention there is provided a compound selected from the group consisting of: 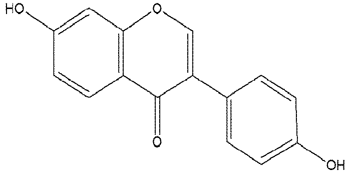 4',7-Dihydroxyisoflavone (Daidzein), 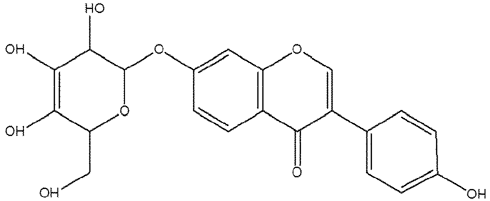 Daidzein 7-glucoside (Daidzin), 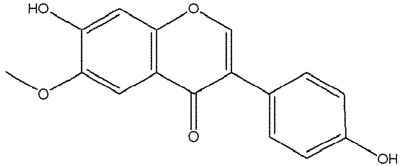 4',7-Dihydroxy-6-methoxyisoflavone (Glycitein),

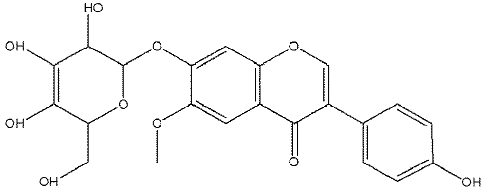

Glycitein 7-glucoside (Glycitin), or combinations thereof for use in treating a retroviral infection in a subject, the treatment further comprising administering a therapy directed to treating the retroviral infection.

Also described is a method of reactivating latent retroviral infections, for example, HIV infection, using isoflavones or pharmaceutically acceptable derivatives thereof, and a method of investigating reactivating mechanisms at the transcriptional level via long terminal repeat (LTR) in different cell models with an effective amount of isoflavones.

Further described are chemical compounds having core structures of isoflavones that are capable of reactivating latent retroviruses from latently infected cells. Methods of using the compounds having the core structures of isoflavones for reactivation and/or treatment of latent retroviral infections are also described.

The methods of reactivating latent retroviral infections in a subject can be implemented alone or in combination with therapies directed to treating the retroviral infection at active stage of viral replication and/or therapies directed to stimulating the subject's immune system.

Other features and advantages of the invention will be apparent from and are encompassed by the following detailed description and claims.

### BRIEF DESCRIPTION OF THE DRAWINGS

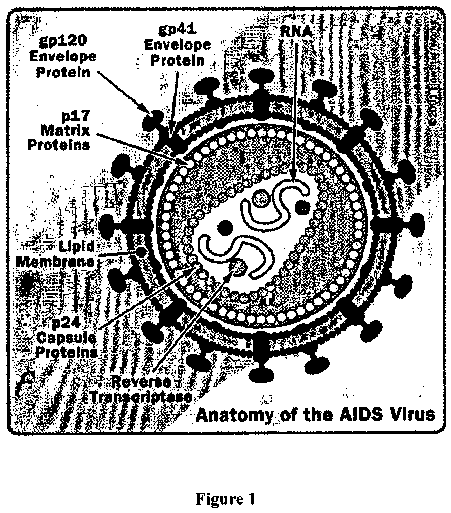

Figure 1. Anatomy of a human immunodeficiency virus (HIV).

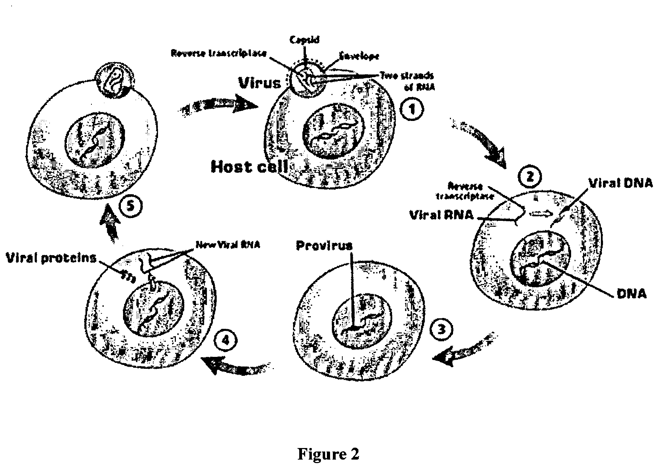

Figure 2. Pro-viral latency in specific long-lived cell types. The presence of replication-competent HIV in resting CD4⁺ T cells, allows the virus to persist for years without evolving despite prolonged exposure to antiretroviral drugs. This latent reservoir of HIV may explain the inability of antiretroviral treatment to cure HIV infection.

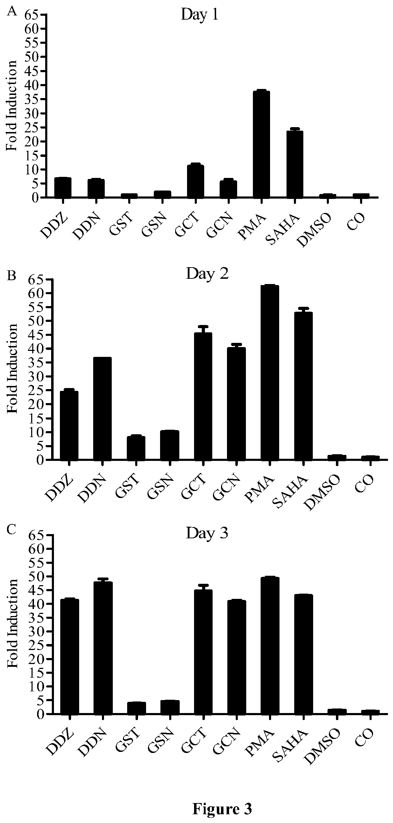

Figure 3. Reactivating ability of isoflavones in latently infected cells. Figure 3A shows the results of p24 production in the supernatant on day 1 of a latently HIV-1 infected cell model, J-Lat full length clone 10.6, treated with 50µg/ml DDZ, DDN, GST, GSN, GCT and GCN, respectively. Figure 3B shows the results of p24 production in the supernatant on day 2 of a latently HIV-1 infected cell model, J-Lat full length clone 10.6, treated with 50µg/ml DDZ, DDN, GST, GSN, GCT and GCN, respectively. Figure 3C shows the results of p24 production in the supernatant on day 3 of a latently HIV-1 infected cell model, J-Lat full length clone 10.6, treated with 50µg/ml DDZ, DDN, GST, GSN, GCT and GCN, respectively. 10µM suberoylanilide hydroxamic acid (SAHA) and 125ng/ml phorbol myristate acetate (PMA) were used as positive controls and 0.25% DMSO as a negative control. All the results are presented as fold induction compared with untreated cells.

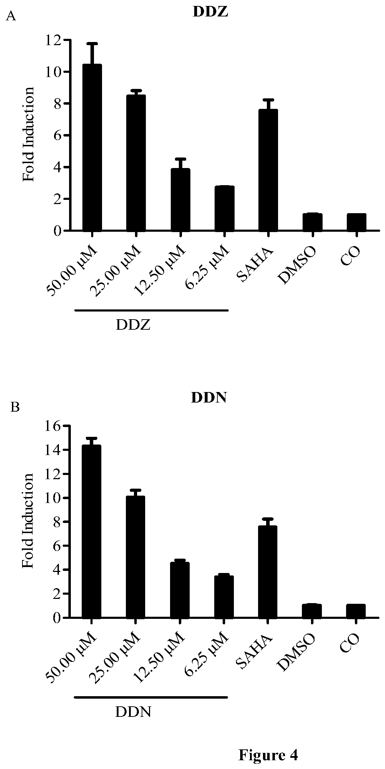

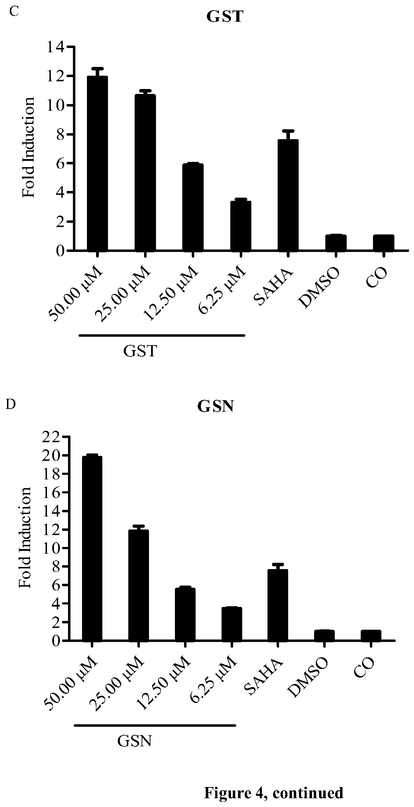

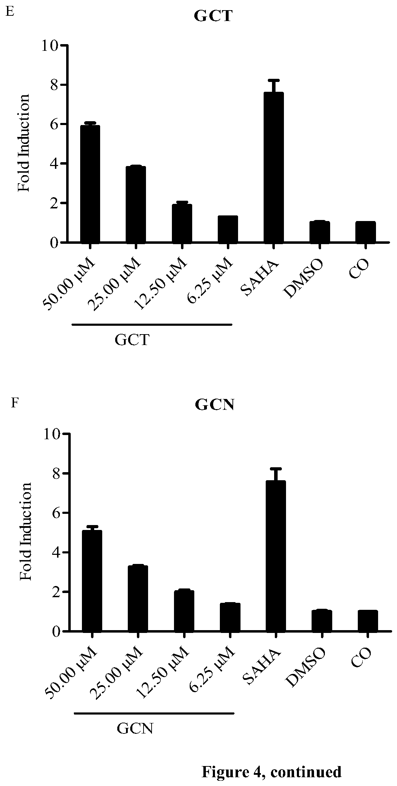

Figure 4. Isoflavone-induced stimulation in TZM-B1 cells. Figure 4A shows the stimulating ability of DDZ in TZM-B1 cells. Figure 4B shows the stimulating ability of DDN in TZM-B1 cells. Figure 4C shows the stimulating ability of GST in TZM-B1 cells. Figure 4D shows the stimulating ability of GSN in TZM-B1 cells. Figure 2E shows the stimulating ability of GCT in TZM-B1 cells. Figure 4F shows the stimulating ability of GCN in TZM-B1 cells. TZM-B1 cells were co-incubated with serially diluted DDZ, DDN, GST, GSN, GCT or GCN for 48hrs. Cells were lysed and luciferase expression level was measured. 10µM SAHA and 0.25% DMSO were used as positive and negative controls, respectively. All the results are presented as fold induction compared with untreated cells.

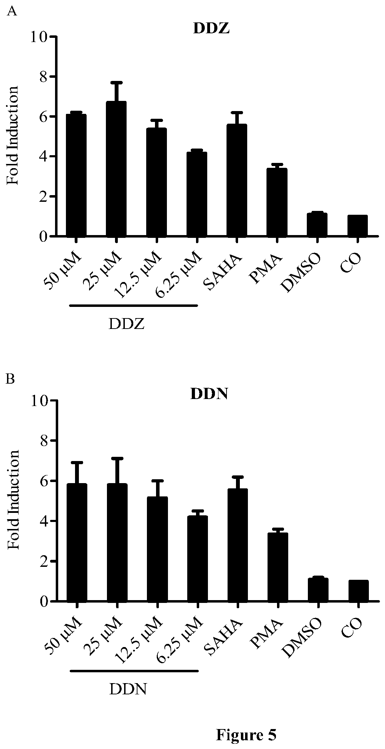

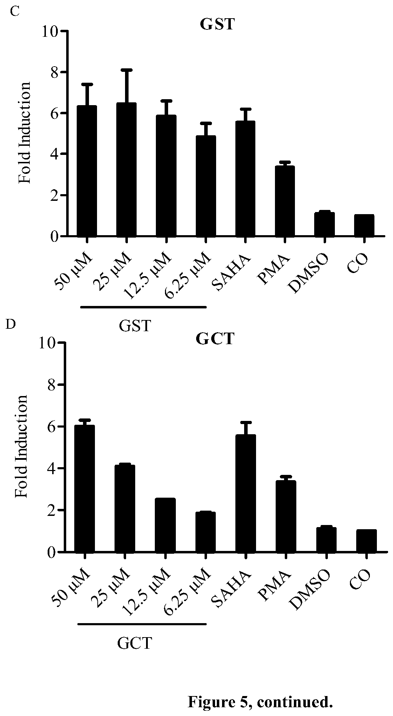

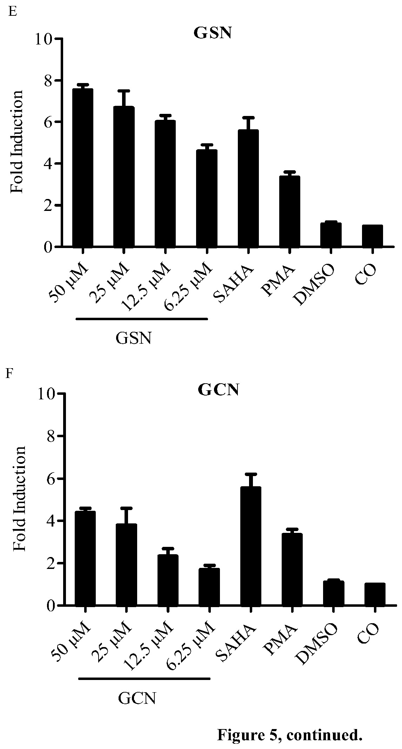

Figure 5. Stimulating ability of isoflavones in 293T cells transfected with pLTR-Luc. 293T cells transfected with pLTR-Luc were co-incubated with serially diluted DDZ, DDN, GST, GSN, GCT or GCN for 48hrs. Cells were lysed and luciferase expression level was measured. 10µM SAHA and 125ng/ml PMA were used as positive controls and 0.25% DMSO was used as a negative control. All results are presented as fold induction compared with untreated cells.

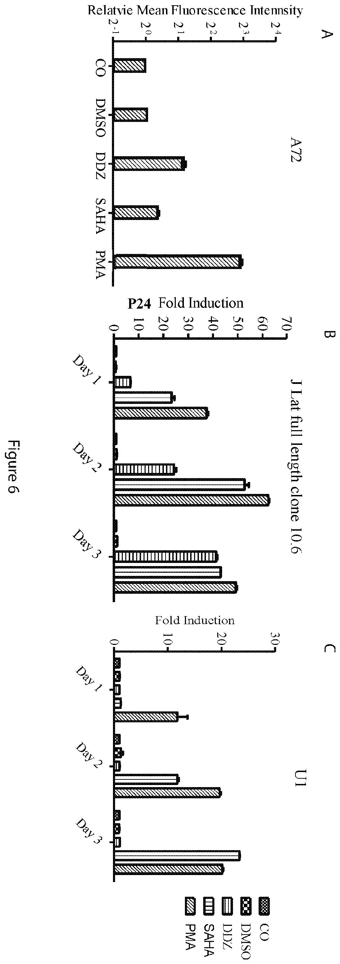

Figure 6. Daidzein reactivates HIV from latently infected T cells. A72 (A), J lat (B) or Ul (C) were stimulated with 50ug/ml Daidzein, or 10 µM SAHA and 125ng/ml PMA as positive control and 0.25% DMSO as negative control for 3 days. 6A shows GFP expression using flow cytometry after 3 days treatment. B and C show viral replication measured in the supernatant of J Lat and U1 cells using p24 ELISA after 1, 2, and 3 days treatment. All results are presented as fold induction compared with the unstimulated control.

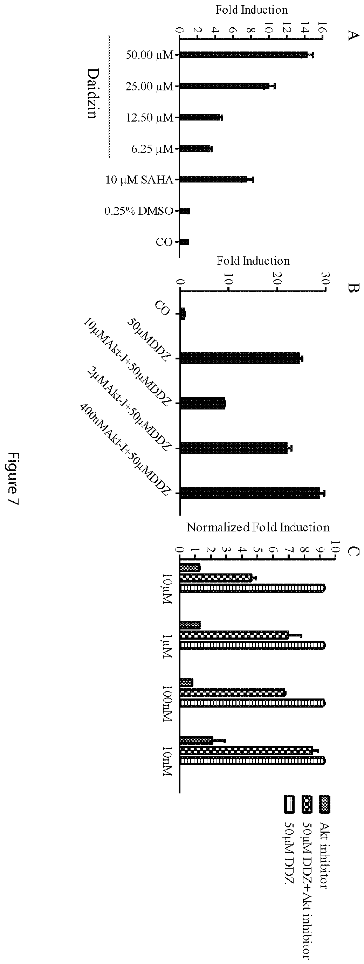

Figure 7. Daidzein activates HIV replication at the level of transcription. TZM-B1 cells were stimulated with increasing concentrations of Daidzein for 72 h. Cells were lysed, and luciferase activity was measured. All results are presented as fold induction compared with the unstimulated control. B, J Lat and C, TZM-Bl cells were pre-incubated, or not, with Akt inhibitor, or treated with Daidzein (50 µM) or not, and viral replication was assessed using p24 ELISA.

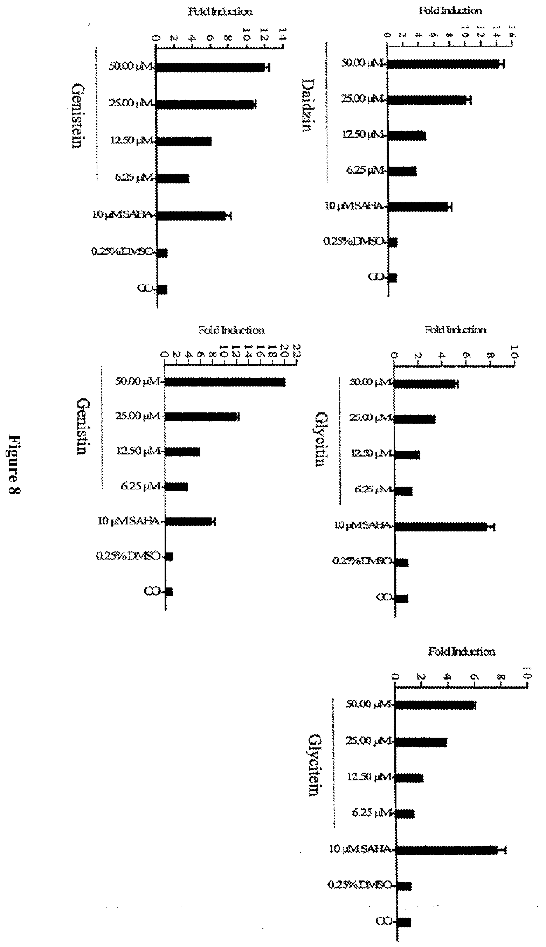

Figure 8. Analogues of Daidzein activate HIV LTR. Serially diluted compounds were co-cultured with TZM-B1 cells in 96 well plates. PMA and SAHA were used as positive control, and solvent-DMSO was used as negative control. Cells were lysed after treatment for 2 days and tested for luciferase activity.

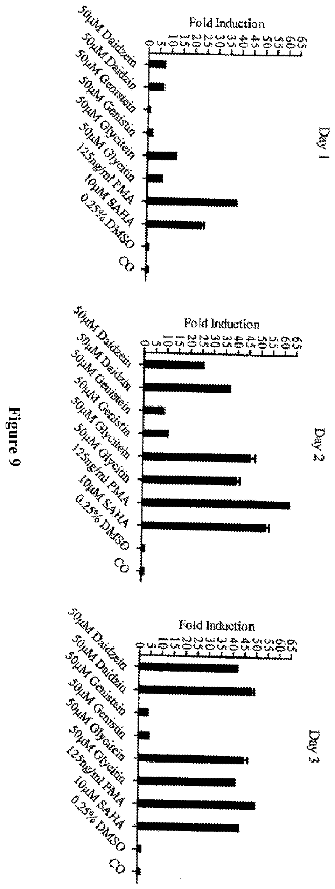

Figure 9. Daidzein and its analogues activate HIV replication in the latency cell model, J Lat full length clone 10.6. Serially diluted compounds were co-cultured with cells in 96 well plates. PMA and SAHA were used as positive control, solvent DMSO was used as negative control. Cell supernatants were harvested for p24 test after treatment for day 1(A), day 2(B) and day 3(C).

### DETAILED DISCLOSURE OF THE INVENTION

Several aspects of the invention are described below, with reference to examples for illustrative purposes only. It should be understood that numerous specific details, relationships, and methods are set forth to provide a full understanding of the invention. One having ordinary skill in the relevant art, however, will readily recognize that the invention can be practiced without one or more of the specific details or practiced with other methods, protocols, reagents, cell lines and animals. The present invention is not limited by the illustrated ordering of acts or events, as some acts may occur in different orders and/or concurrently with other acts or events. Furthermore, not all illustrated acts, steps or events are required to implement a methodology in accordance with the present invention. Many of the techniques and procedures described, or referenced herein, are well understood and commonly employed using conventional methodology by those skilled in the art.

Prior to setting forth the invention in detail, it may be helpful to the understanding thereof to define several terms, and these are accordingly set forth below. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly used dictionaries, should be interpreted as having a meaning that is consistent with their meaning in the context of the relevant art and/or as otherwise defined herein.

As used herein, the singular forms "a", "an", and "the" include both singular and plural referents unless the context clearly dictates otherwise.

The terms "includes", "including" as used herein are synonymous with "contains", "concluding" or "containing", "contains", and are inclusive or open-ended and do not exclude additional, non-recited members, elements, or method steps. The recitation of numerical ranges by endpoints includes all numbers and fractions subsumed within the respective ranges, as well as the recited endpoints.

As used herein, the term "agent" refers to any chemical (e.g., inorganic or organic), biochemical or biological substance, molecule or macromolecule (e.g., biological macromolecule), a combination, or a mixture thereof, a sample of undetermined composition, or an extract made from biological materials such as bacteria, plants, fungi, animal cells, or tissues. Examples of agents include, but are not limited to, nucleic acids, oligonucleotides, ribozymes, polypeptides, proteins, peptides, peptidomimetics, antibodies, fragments or derivatives of antibodies, aptamers, chemical substances, organic molecules, small organic molecules, lipids, carbohydrates, polysaccharides, etc., and any combinations thereof.

The phrase "therapeutically effective amount" refers to the amount of active ingredients used in the treatment of a retroviral infection, for example HIV infection, according to the current invention. This amount will reduce or eliminate the retroviral infection.

The term "prophylaxis" is related to "prevention," and refers to a measure or procedure the purpose of which is to prevent, rather than to treat or cure a disease. Therefore, the term "prophylactically effective amount" means the amount of the subject compound that will prevent the HIV infection in a subject receiving the prophylactically effective amount of the subject compound.

"Prevention," as used herein, includes but is not limited to, delaying the onset of symptoms, preventing relapse to a disease, or a combination thereof. Prevention as used herein does not require the complete absence of symptoms.

For general methods relating to the invention, reference is made inter alia to well-known textbooks, including, e.g., "Molecular Cloning: A Laboratory Manual."(Sambrook, 2001), Animal Cell Culture (Morgan, 1993), Gene Transfer Vectors for Mammalian Cells (Miller, 1987); " Short Protocols in Molecular Biology, 3nd Ed." (Ausubel, 1992); "Recombinant DNA Methodology II" (Wu, 1995)
The term "retrovirus" is used herein in its conventional meaning and generally encompasses a class of viruses in which the genetic material is single-stranded RNA and which employ reverse transcriptase to transcribe the viral RNA into DNA in a host. Retroviruses as intended herein may particularly belong to the viral family Retroviridae, and more particularly to the subfamily Lentivirinae. Retroviruses as intended herein may be pathogenic (*i.e.,* causing a demonstrable disease phenotype in an infected host) or may be non-pathogenic (*i.e.,* wherein an infected host's condition does not manifest a demonstrable disease phenotype). Particularly intended herein are retroviruses infecting animals, more preferably retroviruses of warm-blooded animals, even more preferably of vertebrate animals, still more preferably of mammals, yet more preferably of primates, and most preferably of humans. Particularly preferred herein are human retroviruses including, but not limited to, Human immunodeficiency virus type 1 (HIV-1), Simian immunodeficiency virus (SIV), Human immunodeficiency virus type 2 (HIV-2), Equine infectious anemia virus (EIAV), Feline immunodeficiency virus (FIV), Visna lentivirus, Bovine immunodeficiency virus (BIV), Simian retrovirus type 2 (SRV-2), Simian retrovirus type 1 (SRV-1), Squirrel monkey retrovirus (SMRV-H), Simian Mason-Pfizer virus (MPMV), Mouse mammary tumor virus (MMTV), Mouse intracisternal a-particle (IAP-MIA14), Hamster intracisternal a-particle (IAP-H18), Rous sarcoma virus (RSV), Bovine leukemia virus (BLV), Human T-cell leukemia virus type II (HTLV-2), Human T-cell leukemia virus type I (HTLV-1), Baboon endogenous virus (BaERV), Gibbon ape leukemia virus (GaLV), Feline leukemia virus (FeLV), Moloney murine leukemia virus (MuLV), Human spumaretrovirus (HSRV), Caprine arthritis encephalitis virus (CAEV), and Human Endogenous Retrovirus (HERV).

Reference to "diseases or conditions associated with a retrovirus" generally encompasses any and all states of a host resulting from the host having been infected with the retrovirus. Such states may include, but are not limited to, the presence of viral biological material in the infected host, *e.g.,* the presence of provirus in the genome of one or more cells of the infected host, and/or the presence of viral nucleic acids, viral proteins, or viral particles in the infected host. Such states may also comprise of stages when the provirus is dormant or latent, pre-clinical stages when virus is produced in the infected host but without demonstrable disease symptoms, as well as clinical stages involving demonstrable disease symptoms, such as for example acquired immunodeficiency syndrome (AIDS) caused by HIV-1 and HIV-2, or adult T-cell leukemia/lymphoma (ATLL) or tropical spastic paraparesis (TSP) caused by HTLV-1.

Reference to the "activity" of a target such as a complex or protein may generally encompass any one or more aspects of the biological activity of the target, such as without limitation any one or more aspects of its biochemical activity, enzymatic activity, signaling activity and/or structural activity, *e.g.,* within a cell, tissue, organ or an organism. Preferably, the activity is a methylation activity.

In an embodiment, the activity of a target, such as a complex or a protein, may be modulated and, in particular, reduced by introducing into or expressing in a cell, tissue, organ, or an organism, a dominant negative variant of the target, *e.g.,* a dominant negative variant of one or more constituents of the complex, or a dominant negative variant of the protein.

Reference to the "level" of a target, such as a complex or protein, may preferably encompass the quantity and/or the availability (*e.g.,* availability for performing its biological activity) of the target, e.g., within a cell, tissue, organ or an organism. For example, the level of a target may be modulated by modulating the target's expression and/or modulating the expressed target. Modulation of the target's expression may be achieved or observed, e.g., at the level of heterogeneous nuclear RNA (hnRNA), precursor mRNA (pre-mRNA), mRNA, or cDNA encoding the target. For example, decreasing the expression of a target may be achieved by methods known in the art, such as, *e.g.,* by transfecting (*e.g.,* by electroporation, lipofection, etc.) or transducing (*e.g.,* using a viral vector) a cell, tissue, organ, or organism with an antisense agent, such as, *e.g.,* antisense DNA or RNA oligonucleotide, a construct encoding the antisense agent, or an RNA interference agent, such as siRNA or shRNA, or a ribozyme or vectors encoding such, etc.

Also, increasing the expression of a target may be achieved by methods known in the art, for example, transfecting (*e.g.,* by electroporation, lipofection, etc.) or transducing (*e.g.,* using a viral vector) a cell, tissue, organ, or organism with a recombinant nucleic acid which encodes the target under the control of regulatory sequences effecting suitable expression level in the cell, tissue, organ, or organism.

The level of the target may be modulated, for example, via alteration of the formation of the target (such as, *e.g.,* folding, or interactions leading to formation of a complex), and/or the stability (e.g., the propensity of complex constituents to associate to a complex or disassociate from a complex), degradation or cellular localization, of the target.

The term "positive control" refers to a group where a phenomenon is expected. That is, they ensure that there is an effect when there should be an effect, by using an experimental treatment that is already known to produce that effect (and then comparing this to the treatment that is being investigated in the experiment). Herein, examples of positive controls are PMA and SAHA.

The term "negative control" refers to a group where no phenomenon is expected. They ensure that there is no effect when there should be no effect. To continue with the example of drug testing, a negative control is a group that has not been administered the drug of interest. This group receives no preparation, a sham preparation (that is, a placebo), an excipient-only (also called vehicle-only) preparation, or the proverbial "sugar pill". The control group should show a negative or null effect. Herein, one example of a negative control is dimethyl sulfoxide (DMSO) which is a polar aprotic solvent that dissolves both polar and nonpolar compounds and is miscible in a wide range of organic solvents as well as water.

The term "pLTR-Luc" refers to a plasmid which contains a HIV long terminal repeat (LTR) promoter and firefly luciferase reporter gene. In one embodiment, the pLTR-Luc is used for investigating the ability of test samples to regulate gene expression from the LTR. pLTR-Luc plasmid is constructed on the basis of a molecular clone vector pVAX1 and HIV backbone pNL4-3 R⁻E⁻Luc⁺. The pLTR-Luc plasmid is mixed with polyethylenimine (PEI) and added to 293T cells for transfection. Test samples are added for stimulation afterwards.

The term "subject" as used herein refers to an individual, which can be a human or a non-human animal, which is infected by a retrovirus.

The Human Immunodeficiency Virus (HIV) is a Lentivirus, part of the family of Retroviridae. It is a single-stranded, positive-sense, diploid, enveloped RNA virus. Once inside a target cell, the viral RNA genome is reverse transcribed into double-stranded DNA through a virally encoded reverse transcriptase that is transported along with the viral genome into the target cell's cytoplasm. Subsequently, the transcribed viral DNA is imported into the target cell's nucleus and is integrated into the nuclear DNA by an integrase (also virally encoded). The latency of the HIV and other lentiviruses is due to their ability to integrate in the host cell genome and stay there in a latent form, *i*.*e*. without replicating.

Thus, the virus avoids detection by the immune system and can stay there for years resulting in a so called "reservoir" of HIV in the infected subject. Once the virus is re-activated, the viral DNA is transcribed producing new RNA genomes and viral proteins that are packaged and released from the cell as new virus particles, which can infect new cells. HIV mainly infects cells of the immune system, thereby weakening the immune response of the infected subject, which leads to its name "immunodeficiency virus".

An HIV-positive subject may develop AIDS when the virus develops the ability to reproduce. In essence, the HIV will attach and destroy the CD4⁺ T-cells, macrophages, and microglial cells. The destruction of T-cells and macrophages will make the subject prone to infections that are normally easily avoided by the subject's immune system. When CD4⁺ T-cell numbers in the subject's blood drop below 200 cells/µl, the cell-mediated immunity is lost, and infections with a variety of opportunistic microbes appear. Common opportunistic infections and tumors, most of which are normally controlled by robust CD4⁺ T cell-mediated immunity then start to affect the subject. When a subject with HIV infection or AIDS is not treated, he can eventually die from otherwise easy to cure infections, due to the impairment of the immune system.

Due to the latent character of HIV, reservoirs of HIV-DNA can continue to exist during the whole life span of the infected subject, without any significant signs, if e.g. controlled by constant antiviral treatment. Stopping the treatment will however eventually result in reactivation of the virus. The infected subject can therefore never be fully freed of the HIV infection.

Two types of HIV have been characterized: HIV-1 and HIV-2. HIV-1 is the virus that was initially discovered and is the most virulent type, is more infective, and is the cause of the majority of HIV infections in the world. HIV-2 is less infective and smaller percentage of people exposed to HIV-2 is infected. HIV-2 is largely confined to West Africa.

The treatment guidelines to treat HIV infections for the United States are set by the U.S. Department of Health and Human Services (DHHS). The current guidelines for adults and adolescents were stated on December 1, 2009. The details of the current guidelines are available at world wide website: aidsinfo.nih.gov/contentfiles/lvguidelines/adultandadolescentgl.pdf, the contents of which are herein incorporated by reference in its entirety. These guidelines may be revised in the future and such changes can be considered in the practice of the current invention.

Highly active anti-retroviral therapy (HAART) is not sufficient to eliminate HIV infection in a subject. The current invention provides compounds and methods that can be used to reactivate latent HIV infections. The re-activated HIV infections in a cell are prone to elimination by the subject's immune system and/or HAART. The current invention can be practiced alone or in combination with other HIV therapies that target HIV infection at an active stage of viral replication, for example, HAART, to achieve elimination of HIV infection in a subject. Accordingly, the current invention provides compounds and methods that may eliminate HIV infection in a subject.

Embodiments of the current invention provide a class of compounds called isoflavones, and derivatives thereof, and their use in activation of latent retroviral infection, for example, latent HIV infection. Isoflavones comprise a class of organic compounds, often naturally occurring, related to the isoflavonoids. Isoflavones of nutritional interest are substituted derivatives of isoflavone, being related to the parent by the replacement of two or three hydrogen atoms with hydroxyl groups. The isoflavones of embodiments of the current invention include, but are not limited to, Daidzein, Daidzin, Genistein, Genistin, Glycitein, Glycitin, and pharmaceutically acceptable derivatives thereof. Pharmaceutically acceptable derivatives include amides, salts, esters, ethers, organic based derivatives, hydrates, or solvates. Additional pharmaceutically acceptable derivatives are well known to a person of ordinary skill in the art and such derivatives are within the purview of this invention.

Isoflavones are produced via a branch of the general phenylpropanoid pathway that produces flavonoid compounds in higher plants. The phenylpropanoid pathway begins from the amino acid phenylalanine, and an intermediate of the pathway, naringenin, is sequentially converted into the isoflavone genistein by two legume-specific enzymes, isoflavone synthase and a dehydratase. Similarly, another intermediate, naringenin chalcone, is converted to the isoflavone daidzein by sequential action of three legume-specific enzymes: chalcone reductase, type II chalcone isomerase, and isoflavone synthase.

Isoflavones of the current invention are provided in Table 1.

**Table 1. Isoflavones that can reactivate a latent retrovirus, for example, latent HIV-1. The isoflavones are DDZ, DDN, GCT, GCN.**

| **Name** | **Structure** |
|---|---|
| Daidzein (DDZ) | 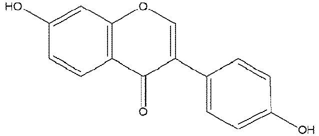 |
| Daidzin (DDN) | 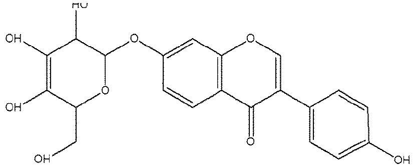 |
| Glycitein (GCT) | 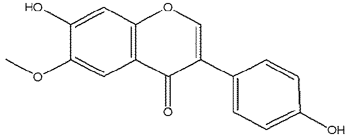 |
| Glycitin (GCN) | 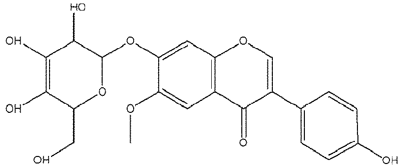 |

Other examples of isoflavones are Genistein (GST) and Genistin (GSN). Also described are class compounds of Formula I and II as described below and use thereof for reactivating latent retroviral infection, for example latent HIV infection. Structures of Formula I and Formula II are provided in Table 2.

**Table 2. Core structures of isoflavones of capable of reactivating latent retroviruses, for example, latent HIV-1.**

| **Name** | **Structure** |
|---|---|
| Formula I | 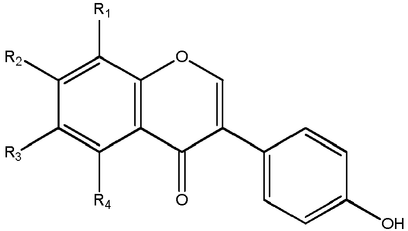 |
| Formula II | 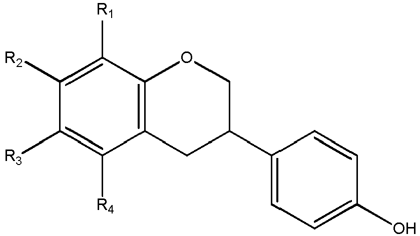 |
| R₁, R₂, R₃, and R₄, independent of each other, can be H, OH, Glycosyl, Halogen, CH₃, OCH₃, CA₃, OCA₃, CN, NO₂, A, or OA; wherein A is a branched or linear alkyl having 1 to 12 C-atoms, wherein one or more H atoms may be replaced by Halogen, OH, OA, COOH, COOA, CN, N(A)². | |

Also described are methods of reactivating latent retroviral infections, for example HIV infection, in a subject. Such methods may include administering to the subject in need of such treatment, a therapeutically effective amount of one or more isoflavones, pharmaceutically acceptable derivatives thereof, or a combination thereof. Further described are methods of reactivating latent retroviral infections, for example HIV infections, in a subject, including administering to the subject in need of such treatment a therapeutically effective amount of compounds having a isoflavone core structure selected from: a) a compound of Formula I: 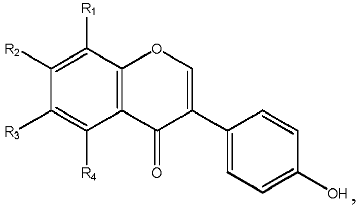 or b) a compound of Formula II: 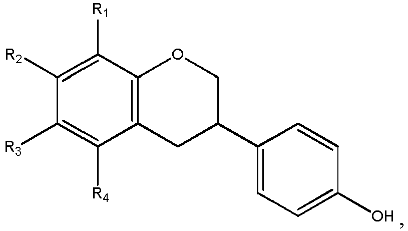 wherein, R₁, R₂, R₃, and R₄, independent of each other, are H, OH, Glycosyl, Halogen, CH₃, OCH₃, CA₃, OCA₃, CN, NO₂, A, or OA, and wherein A is a branched or linear alkyl having 1 to 12 C-atoms, wherein one or more H atoms may be replaced by Hal, OH, OA, COOH, COOA, CN, or N(A)².

Described herein is a method of treating retroviral infection, for example, HIV infection, in a subject by administering to a subject in need of such treatment, a therapeutically effective amount of one or more isoflavones, compounds of Formula I or Formula II, pharmaceutically acceptable derivatives thereof, or a combination thereof. The methods of administering compounds of Formula I or Formula II, pharmaceutically acceptable derivatives thereof, or a combination thereof can be administered alone or in combination with other therapies directed against the retroviral infection at an active stage of viral infection. For example, one method of reactivating HIV infection in a subject by administering compounds of Formula I or Formula II, pharmaceutically acceptable derivatives thereof, or a combination thereof is implemented in combination with HAART.

Methods of treating retroviral infection in a subject by reactivating retroviral infection may be implemented in combination with one or more therapies directed to stimulating the subject's immune system. Stimulating the subject's immune system can facilitate the subject in more robustly fighting infections, for example, reactivated HIV infections and/or other microbial infections.

Non-limiting examples of therapies directed to stimulating the subject's immune system include dendritic cell-based immunotherapy, T-cell adoptive transfer, autologous immune enhancement therapy, genetically engineered T-cells, immune recovery, and/or vaccination. Routine administration of these therapies and/or the modifications directed to treating HIV infections are well known to a person of ordinary skill in the art and such routine administrations and/or modifications are within the purview of the current invention.

Non-limiting examples of therapies directed to stimulating a subject's immune system include administering one or more cytokines to the subject. Such cytokines include, but are not limited to, interleukin-2, interleukin-4, interleukin-5, interleukin-7, interleukin-10, transforming growth factor-α, interferon-y, and combinations thereof.

Therapies directed to stimulating a subject's immune system can comprise of administering small molecule compounds, herbal extracts, and/or combination thereof capable of stimulating the immune system.

Highly active anti-retroviral therapy (HAART): Antiretroviral therapy (ART) is the treatment of people infected with human immunodeficiency virus (HIV) using anti-HIV drugs. Standard treatments consist of a combination of at least three drugs (often called "highly active antiretroviral therapy" or HAART) that suppress HIV replication. Three drugs are used in order to reduce the likelihood of the virus developing resistance. ART has the potential both to reduce mortality and morbidity rates among HIV-infected people, and to improve their quality of life. HAART decreases the subject's total burden of HIV, maintains function of the immune system, and prevents opportunistic infections that often lead to death.

The American National Institutes of Health and other organizations recommend offering antiretroviral treatment to all subjects with AIDS. Because of the complexity of selecting and following a regimen, the severity of the side-effects, and the importance of compliance to prevent viral resistance, such organizations emphasize the importance of involving subjects in therapy choices and recommend analyzing the risks and the potential benefits to subjects with low viral loads.

The advent of HAART has been dated to the 11th International Conference on AIDS in Vancouver, British Columbia, July 7-16, 1996. During that Conference, David Ho, MD, of the Aaron Diamond AIDS Research Center, New York, NY, and George Shaw, MD, PhD, of the University of Alabama at Birmingham School of Medicine, presented viral dynamics data showing that the average person with HIV infection produced 10 billion virions/day, bringing into sharp focus the fact that this was a viral infection that required antiviral treatment. The conference was followed by sequential publications in The New England Journal of Medicine by Hammer and colleagues and Gulick and co-investigators illustrating the substantial benefit of indinavir-based HAART. This concept of 3-drug therapy was quickly incorporated into clinical practice and rapidly showed impressive benefit with a 60% to 80% decline in rates of AIDS, death, and hospitalization.

There are several classes of drugs, which are usually used in combination, to treat HIV infection. Use of these drugs in combination is generally termed ART, or Anti-Retroviral Therapy. Anti-retroviral (ARV) drugs are broadly classified by the phase of the retrovirus life-cycle that the drug inhibits. Typical combinations include two NRTIs (Nucleoside Reverse Transcriptase Inhibitors) and one PI (Protease Inhibitor) or two NRTIs and one NNRTI (Non-Nucleoside Reverse Transcriptase Inhibitor). Combinations of antiretrovirals are subject to positive and negative synergies which limit the number of useful combinations.

Entry inhibitors (or fusion inhibitors) interfere with binding, fusion and entry of HIV-1 to the host cell by blocking one of several targets. Maraviroc and enfuvirtide are two agents in this class. Maraviroc works by targeting CCR5, a co-receptor located on human helper T-cells. Caution should be used when administering this drug due to a possible shift in tropism that allows HIV to target an alternative co-receptor such as CXCR4. In rare cases, individuals may have a mutation in the CCR5 delta gene resulting in a nonfunctional CCR5 co-receptor and in turn, a means of resistance or slow progression of the disease. However, as mentioned previously, this can be overcome if an HIV variant that targets CXCR4 becomes dominant. To prevent fusion of the virus with the host membrane, Fuzeon (T20) can be used. Fuzeon is a peptide drug that must be injected and acts by interacting with the N-terminal heptad repeat of gp41 of HIV to form an inactive hetero six-helix bundle, therefore preventing infection of host cells.

Nucleoside reverse transcriptase inhibitors (NRTI) and nucleotide reverse transciptase inhibitors (NtRTI) are nucleoside and nucleotide analogues that inhibit reverse transcription. NRTIs are chain terminators that, once incorporated, work by preventing other nucleosides from also being incorporated because of the absence of a 3' OH group; they both act as competitive substrate inhibitors. Non-limiting examples of NRTIs include deoxythymidine, zidovudine, stavudine, didanosine, zalcitabine, abacavir, lamivudine, emtricitabine, and tenofovir.

Non-Nucleoside reverse transcriptase inhibitors (NNRTI) inhibit reverse transcriptase by binding to an allosteric site of the enzyme; NNRTIs act as non-competitive inhibitors of reverse transcriptase. NNRTIs affect the handling of substrate (nucleotides) by reverse transcriptase by binding near the active site and causing "molecular arthritis." NNRTIs can be further classified into 1st generation and 2nd generation NNRTIs. 1st generation NNRTIs are more rigid in structure and resistance can quickly be developed against them; However, 2nd generation NNRTIs have a more flexible structure and can adjust more readily and resist mutation more effectively. NNRTIs, for example, include nevirapine, delavirdine, efavirenz, and rilpivirine.

Integrase inhibitors inhibit the enzyme integrase, which is responsible for integration of viral DNA into the DNA of the infected cell. There are several integrase inhibitors currently under clinical trial, and raltegravir became the first to receive FDA approval in October 2007. Raltegravir has two metal binding groups that compete for substrate with two Mg²⁺ ions at the metal binding site of integrase. Another clinically approved integrase inhibitor is Elvitegravir.

Protease inhibitors block the viral protease enzyme necessary to produce mature virions upon budding from the host membrane. Particularly, these drugs prevent the cleavage of gag and gag/pol precursor proteins. Virus particles produced in the presence of protease inhibitors are defective and mostly non-infectious. Non-limiting examples of HIV protease inhibitors are Lopinavir, Indinavir, Nelfinavir, Amprenavir, and Ritonavir. Maturation inhibitors have a similar effect by binding to gag, but development of two experimental drugs in this class, Bevirimat and Vivecon, was halted in 2010. Resistance to some protease inhibitors is high. Second generation drugs have been developed that are effective against otherwise resistant HIV variants.

When antiretroviral drugs are used improperly, these multi-drug resistant strains can become the dominant genotypes very rapidly. Improper serial use of the reverse transcriptase inhibitors zidovudine, didanosine, zalcitabine, stavudine, and lamivudine can lead to the development of multi-drug resistant mutations. The mutations can include the V75I, F77L, K103N, F116Y, Q151M, and the M184V mutation. These mutations were observed before protease inhibitors had come into widespread use. The mutants retained sensitivity to the early protease inhibitor saquinavir. These mutants were also sensitive to the rarely used reverse transcriptase inhibitor foscarnet.

In recent years, drug companies have worked together to combine these complex regimens into simpler formulas, termed fixed-dose combinations. For instance, two pills containing two or three medications each can be taken twice daily. This greatly increases the ease with which they can be taken, which in turn increases adherence, and thus their effectiveness over the long-term. Lack of adherence is a cause of resistance development in medication-experienced subjects. Subjects who maintain proper therapy can stay on one regimen without developing resistance. This greatly increases life expectancy and leaves more drugs available to the individual should the need arise.

Examples of HAART regimen that can be administered along with compounds of the current invention include, but are not limited to, tenofovir/emtricitabine (a combination of two NRTIs) and efavirenz (a NNRTI); tenofovir/emtricitabine and raltegravir (an integrase inhibitor); tenofovir/emtricitabine, ritonavir, and darunavir (both latter are protease inhibitors); and tenofovir/emtricitabine, ritonavir, and atazanavir (both latter are protease inhibitors).

Methods of treating retroviral infection in a subject by reactivating retroviral infection can also be implemented in combination with one or more therapies directed to treating the retroviral infection. The therapy directed to treating the retroviral infection includes administering one or more drugs selected from the group consisting of nucleoside reverse transcriptase inhibitors, non-nucleoside reverse transcriptase inhibitors, protease inhibitors, integrase inhibitors, and combinations thereof. Nucleoside reverse transcriptase inhibitors useful in embodiments of the present invention include tenofovir, emtricitabine, and combinations thereof. A non-nucleoside reverse transcriptase inhibitor useful in embodiments of the present invention incudes efavirenz. Protease inhibitors useful in embodiments of the present invention include ritonavir, darunavir, atazanavir, and combinations thereof. An integrase inhibitor useful in embodiments of the present invention incudes raltegravir. The therapy directed to treating the retroviral infection may include administering two nucleoside reverse transcriptase inhibitors and one protease inhibitor, or alternatively, two nucleoside reverse transcriptase inhibitors and one non-nucleoside reverse transcriptase inhibitor.

The pharmaceutical compositions for use in accordance with embodiments of the present invention, may include, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be any conventional route of systemic administration. Preferably the agent is administered orally. Other routes of administration that can be used in accordance with this invention include rectally, parenterally, by injection (*e.g.* intravenous, subcutaneous, intramuscular or intraperitioneal injection), or nasally.

For injection, the active ingredient can be in the form of a parenterally acceptable aqueous solution which is pyrogen-free and has suitable pH, isotonicity and stability. Preservatives, stabilizers, buffers, antioxidants and/or other additives may be included, as required.

Compositions may be formulated in liquid or solid forms. Solid forms may be provided for reconstitution prior to administration by intravenous or subcutaneous injection, or for inhalation. The composition employed will depend on the physicochemical properties of the molecule and the route of delivery. Formulations may include excipients, or combinations of excipients, for example: sugars, amino acids and surfactants. Liquid formulations may include a wide range of antibody concentrations and pH. Solid formulations may be produced by lyophilisation, spray drying, or drying by supercritical fluid technology, for example. Formulations will depend upon the intended route of delivery; for example, formulations for pulmonary delivery may consist of particles with physical properties that ensure penetration into the deep lung upon inhalation.

### Materials and Methods:

### Cell lines

The following reagent was obtained through the AIDS Research and Reference Reagent Program, Division of AIDS, NIAID, National Institute of Health: J Lat full length clone 10.6, A72 and U1. Cells were grown in RPMI 1640 medium containing penicillin (100 IU/ml), streptomycin (100 IU/ml), and 10% fetal bovine serum at 37°C with 5% CO₂. TZM-B1 cells were cultured in Dulbecco's modified Eagle's medium containing the same supplements.

### Luciferase Assay

TZM-B1 cells (10⁶ cells) were lysed in 200 µL of luciferase reporter lysis buffer (catalog number E387A, Promega, Madison, WI). After centrifugation, cell debris was discarded. Half of the lysis solution was used for analysis using a luminometer.

### Miscellaneous Methods

p24 ELISA assay, plasmid construction, and cell culture were performed using standard methodologies. The reactivation tests were carried out in cultures of latently infected cell line, J-Lat full length clone 10.6, TZM-B1 or 293T cells.

The HIV-1 p24 production was assessed by ZeptoMetrix HIV-1 p24 Antigen ELISA kit, and luciferase activity was assessed by Promega Luciferase Assay System kit). The molecular structures were drawn by CambridgeSoft ChemDraw software.

### EXAMPLE 1: EFFECT OF ISOFLAVONES ON HIV-1 GENE EXPRESSION

Isoflavones, such as daidzein, increase p24 production in the supernatant of latently infected cell line, J-Lat full length clone 10.6. J-Lat full length clone 10.6 cells were seeded in 96-well U bottom plates at 1 x 10⁵/well in 100 µl growth medium, followed by treatment of 50 µg/ml DDZ, DDN, GST, GSN, GCT, or GCN. The supernatant was collected on day 1, 2, and 3 for HIV-1 p24 test after co-incubation. 10 µM SAHA and 125 ng/ml PMA were used as positive controls; whereas, 0.25% DMSO was used as a negative control. The results obtained with untreated cells (marked as CO) were set at a value of 1. All the results are presented as fold induction compared to untreated cells.

The results are depicted in Figure 3, showing a significant increase of p24 production in the supernatant in isoflavone-treated cells versus untreated cells. In addition, with the time of co-incubation, a clear increase of p24 production was also observed in the supernatant of isoflavone-treated cells as shown in Figure 3A-3C.

### EXAMPLE 2: EFFECT OF ISOFLAVONES ON REGULATION OF GENE EXPRESSION FROM HIV LONG TERMINAL REPEAT IN TXM-B 1 CELL MODEL

In this experiment, it is shown that isoflavones, such as daidzein, increase gene expression from LTR. To test the activating ability in TZM-B1 cell model, TZM-B1 cells were seeded in 96-well flat bottom plates at 1.3 x 10⁵/well in 100 µl growth medium. After overnight culture, TZM-Bl cells were co-incubated with serially diluted DDZ, DDN, GST, GSN, GCT or GCN for 48hrs. After 36 hr co-incubation, the supernatant was discarded. The cells were lysed by 50 µl lysis buffer and kept at room temperature for 30 min. 40 µl lysate was transferred into a white plate for luciferase assay. 10 µM SAHA and 0.25% DMSO were used as positive control and negative control, respectively. The result obtained with untreated cells (marked as CO) was set at a value of 1. All the results are presented as fold induction compared with untreated cells.

The results are depicted in Figure 4, showing an obvious increase of luciferase activity in isoflavone-treated cells versus untreated cells. In addition, the increase of transcription from the HIV LTR was dose-dependent in isoflavone-treated cells, as shown in Figure 4A-4F.

### EXAMPLE 3: EFFECT OF ISOFLAVONES ON REGULATION OF GENE EXPRESSION FROM HIV LONG TERMINAL REPEAT (LTR) IN 293T CELLS TRANSFECTED WITH pLTR-Luc

In this experiment, it is shown that isoflavones, such as daidzein, increase gene expression from LTR. 293T cells were seeded in 24-well plates at 1 x 10⁵/well in 500 µl growth medium. After overnight culture, the medium was replaced with fresh culture medium. The pLTR-Luc plasmid was mixed with PEI and kept at room temperature for 15 min. Afterwards, the mixture was added to 293T cells. After 6 hr co-incubation, the medium was aspirated and transfected 293T cells were co-incubated with serially diluted DDZ, DDN, GST, GSN, GCT or GCN for 48hrs. After 36hr co-incubation, the supernatant was discarded. The cells were lysed by 200 µl lysis buffer and kept at room temperature for 30min. 150 µl lysate was transferred into a white plate for luciferase assay. 10 µM SAHA and 125ng/ml PMA were used as positive control and 0.25% DMSO as negative control, respectively. The result obtained with untreated cells (marked as CO) was set at a value of 1. All the results are presented as fold induction compared with untreated cells.

The results are depicted in Figure 5, showing an increase of luciferase activity in isoflavone-treated cells versus untreated cells. In addition, the increase of transcription from the HIV LTR was dose-dependent in isoflavone-treated cells, as shown in Figure 5A-5F.

### EXAMPLE 4 - ACTIVATION OF HIV PRODUCTION IN HJV CHRONICALLY INFECTED CELL LINES

Ul or J Lat cells were plated at 1 x 10⁵ cells/ml in 96-well plates; supernatant was collected before stimulation with compounds daily, and viral release in the supernatant was quantified by p24 ELISA (PerkinElmer Life Sciences) or flow cytometry for GFP detection. Base-line levels were 50-100 pg/ml in control cells. Pre-treatment with inhibitor was done 1 h before stimulation by tested compounds.

### Daidein reactivate IDV replication from a latently infected T cell line

To characterize the ability of Daidzein to stimulate viral replication, 50 µM of tested compound was used to stimulate chronically infected cell lines. The production of viral particles was analyzed from J Lat and Ul cells using p24 ELISA and GFP expression from A72 cells using flow cytometry. HIV reactivation was observed in lymphocytic A72 cells treated with Daidzein and PMA but not SAHA or DMSO (Fig. 6A). About 50 fold HIV induction was detected in lymphocytic J Lat full length clone 10.6 treated with Daidzein, SAHA and PMA (Fig 6B). SAHA reactivated HIV replication in Ul cells as expected;d however, Daidzein treatment of Ul cells did not induce HIV replication (Fig 6 C). This result suggested that Daidzein reactivate HIV latency fron T cells with a comparable potency of SAHA and likely through a distinct mechanism from SAHA.

### Daidzein activates HIV replication at the level of transcription

To determine whether Daidzein stimulates viral replication by affecting gene expression from an HIV LTR, TZM-B1 cells that contain an integrated HIV LTR linked to the luciferase reporter gene in their genome were utilized. Daidzein induced a dose-dependent increase in luciferase activity starting at concentration of 6.25 µM, and reached 14 fold induction of HIV replication compared with the unstimulated control in these cells at concentration of 50 µM (Fig. 7A).

### Daidzein-induced IDV replication is dependent on the Akt signaling pathway

The Akt pathway is involved in SAHA-induced HIV replication. To determine whether Daidzein also activates this pathway, Akt inhibitors were utilized in a TZM-B1 and J Lat cell based assay, respectively. Akt inhibitors decreased Daidzein-induced viral replication by more than 50% (Fig. 7B, compare lanes 2-3). Importantly, these inhibitors had no significant effect on basal levels of HIV production (Fig. 7C).

These results suggest that Daidzein stimulates viral replication in cells in an Akt-dependent manner.

### EXAMPLE 5 - STRUCTURE-ACTIVITY RELATIONSHIPS OF DAIDZEIN AND ITS ANALOGS

To acquire the structure-activity relationships (SARs) and understand the activity requirements by substituents on privileged structure, five analogues of Daidzein were tested in the TZM-B1 cell line (Fig. 8) and J Lat (Fig. 9). All of the 5 analogues tested were able to activate LTR in the TZM-B1 cell line with comparable potency. However, genistein and genistin did not show potent activity to reactivate HIV replication in the latently infected T cell line J Lat, whereas the other four analogues Daidzein, Daizin, Glycitein and Glycitin potently reactivated HIV replication at a concentration of 50 µM, with induced HIV replication for 41-, 48-, 44-, 40-fold compared with unstimulated cells, respectively.

Analysis of the SARs of the six compounds demonstrated that the bio functional core structure is 4'-hydroxyisoflavone (Table 2). Addition of a hydroxyl group at the C-5 position significantly decreases its bio-function. Neither glycosylation of the hydroxyl group at C-7 position or methoxyl modification at the C-6 position has a significant effect on its bio function (Table. 1).

The foregoing description of the specific embodiments should fully reveal the general nature of the invention so that others can, by applying knowledge within the skill of the art, readily modify and/or adapt for various applications such specific embodiments, without undue experimentation, without departing from the general concept of the present invention. Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents. Moreover, the breadth and scope of the present invention should not be limited by any of the above-described exemplary embodiments, but should similarly be defined only in accordance with the following claims and their equivalents.

### REFERENCES

1. Ausubel, F. M. (1992). Short Protocols in Molecular Biology: Wiley.
2. Barresinoussi, F., Chermann, J. C., Rey, F., Nugeyre, M. T., Chamaret, S., Gruest, J., ... Montagnier, L. (1983). Isolation of a T-Lymphotropic Retrovirus from a Subject at Risk for Acquired Immune-Deficiency Syndrome (Aids). Science, 220(4599), 868-871. doi: DOI 10.1126/science.6189183
3. Chun, T. W., Stuyver, L., Mizell, S. B., Ehler, L. A., Mican, J. A., Baseler, M., ... Fauci, A. S. (1997). Presence of an inducible HIV-1 latent reservoir during highly active antiretroviral therapy. Proc Natl Acad Sci U S A, 94(24), 13193-13197.
4. Contreras, X., Schweneker, M., Chen, C. S., McCune, J. M., Deeks, S. G., Martin, J., & Peterlin, B. M. (2009). Suberoylanilide hydroxamic acid reactivates HIV from latently infected cells. J Biol Chem, 284(11), 6782-6789. doi: 10.1074/jbc.M807898200
5. Finzi, D., Blankson, J., Siliciano, J. D., Margolick, J. B., Chadwick, K., Pierson, T., ... Siliciano, R. F. (1999). Latent infection of CD4+ T cells provides a mechanism for lifelong persistence of HIV-1, even in subjects on effective combination therapy. Nature Medicine, 5(5), 512-517. doi: 10.1038/8394
6. Finzi, D., Hermankova, M., Pierson, T., Carruth, L. M., Buck, C., Chaisson, R. E., ... Siliciano, R. F. (1997). Identification of a reservoir for HIV-1 in subjects on highly active antiretroviral therapy. Science, 278(5341), 1295-1300.
7. Folks, T., Powell, D. M., Lightfoote, M. M., Benn, S., Martin, M. A., & Fauci, A. S. (1986). Induction of HTLV-III/LAV from a nonvirus-producing T-cell line: implications for latency. Science, 231(4738), 600-602.
8. Graci, J. D., Colacino, J. M., Peltz, S. W., Dougherty, J. P., & Gu, Z. (2009). HIV type-1 latency: targeted induction of proviral reservoirs. Antivir Chem Chemother, 19(5), 177-187.
9. Miller, J. H. (1987). Gene transfer vectors for mammalian cells: Cold Spring Harbor Laboratory Press.
10. Morgan, S. J., & Darling, D. C. (1993). Animal cell culture: BIOS Scientific Publishers.
11. Sambrook, J., Russell, D. W., & Russell, D. W.. (2001). Molecular cloning: a laboratory manual: Cold Spring Harbor, NY: Cold Spring Harbor Laboratory.
12. Siliciano, J. D., Kajdas, J., Finzi, D., Quinn, T. C., Chadwick, K., Margolick, J. B., ... Siliciano, R. F. (2003). Long-term follow-up studies confirm the stability of the latent reservoir for HIV-1 in resting CD4+ T cells. Nature Medicine, 9(6), 727-728. doi: 10.1038/nm880
13. Siliciano, R. F., & Greene, W. C. (2011). HIV latency. Cold Spring Harb Perspect Med, 1(1), a007096. doi: 10.1101/cshperspect.a007096
14. Strain, M. C., Gunthard, H. F., Havlir, D. V., Ignacio, C. C., Smith, D. M., Leigh-Brown, A. J., ... Wong, J. K. (2003). Heterogeneous clearance rates of long-lived lymphocytes infected with HIV: intrinsic stability predicts lifelong persistence. Proc Natl Acad Sci U S A, 100(8), 4819-4824. doi: 10.1073/pnas.0736332100
15. UNAIDS. (2012). UNAIDS Report on the Global AIDS Epidemic.
16. Wong, J. K., Hezareh, M., Gunthard, H. F., Havlir, D. V., Ignacio, C. C., Spina, C. A., & Richman, D. D. (1997). Recovery of replication-competent HIV despite prolonged suppression of plasma viremia. Science, 278(5341), 1291-1295.
17. Wu, R. (1995). Recombinant DNA Methodology II: Academic Press.

## Claims

1. A compound selected from the group consisting of: 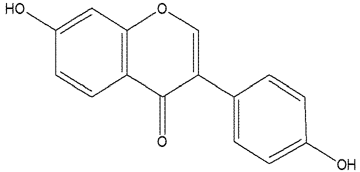 4',7-Dihydroxyisoflavone (Daidzein), 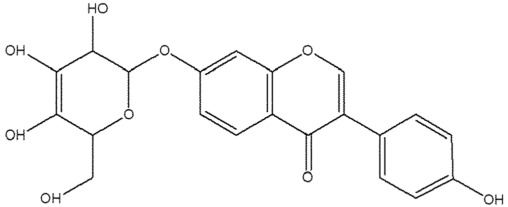 Daidzein 7-glucoside (Daidzin), 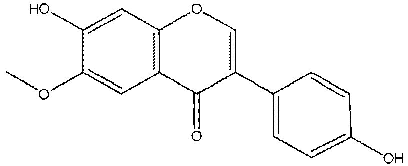 4',7-Dihydroxy-6-methoxyisoflavone (Glycitein), 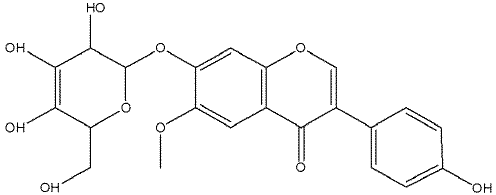 Glycitein 7-glucoside (Glycitin), or combinations thereof for use in reactivating a latent retroviral infection in a subject.

2. A compound selected from the group consisting of: 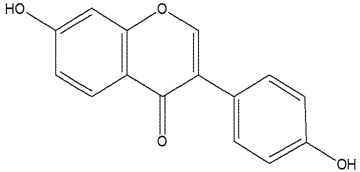 4',7-Dihydroxyisoflavone (Daidzein), 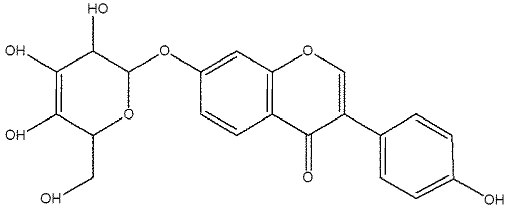 Daidzein 7-glucoside (Daidzin), 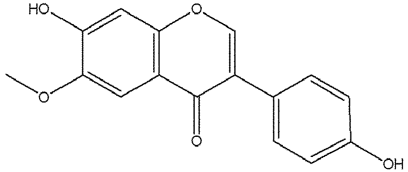 4',7-Dihydroxy-6-methoxyisoflavone (Glycitein), 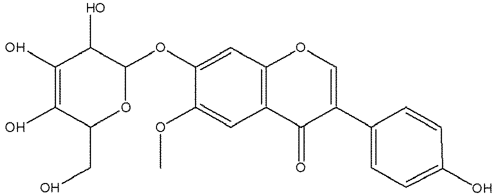 Glycitein 7-glucoside (Glycitin), or combinations thereof,
for use in treating a retroviral infection in a subject, the treatment further comprising administering a therapy directed to stimulating the immune system of the subject.

3. A compound selected from the group consisting of: 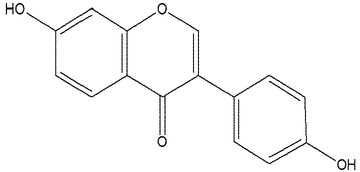 4',7-Dihydroxyisoflavone (Daidzein), 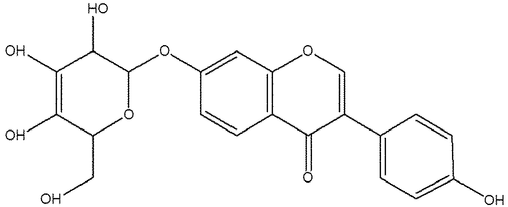 Daidzein 7-glucoside (Daidzin), 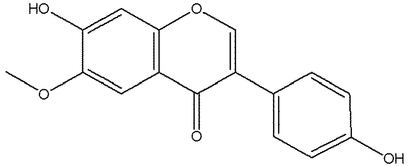 4',7-Dihydroxy-6-methoxyisoflavone (Glycitein), 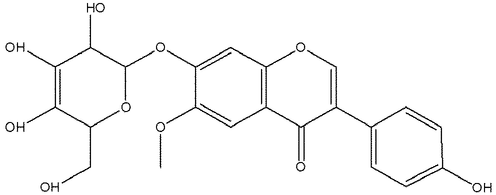 Glycitein 7-glucoside (Glycitin), or combinations thereof for use in treating a retroviral infection in a subject, the treatment further comprising administering a therapy directed to treating the retroviral infection.

4. A compound for use according to claim 2, wherein the therapy directed to stimulating the immune system of the subject comprises dendritic cell-based immunotherapy, T-cell adoptive transfer, autologous immune enhancement therapy, genetically engineered T-cells, immune recovery, vaccination, or combinations thereof.

5. A compound for use according to claim 2, wherein the therapy directed to stimulating the immune system of the subject includes administering one or more cytokines to the subj ect.

6. A compound for use according to claim 5, wherein the one or more cytokines are selected from the group consisting of interleukin-2, interleukin-4, interleukin-5, interleukin-7, interleukin-10, transforming growth factor-α, interferon-y, and a combination thereof.

7. A compound for use according to any preceding claim, wherein the latent retroviral infection is human immunodeficiency virus (HIV) infection.

8. A compound for use according to claim 3, wherein the therapy directed to treating the retroviral infection comprises administering at least one nucleoside reverse transcriptase inhibitor, wherein the at least one nucleoside reverse transcriptase inhibitor is preferably selected from the group consisting of tenofovir, emtricitabine, and combinations thereof.

9. A compound for use according to claim 3, wherein the therapy directed to treating the retroviral infection comprises administering at least one non-nucleoside reverse transcriptase inhibitor, wherein the at least one non-nucleoside reverse transcriptase inhibitor is preferably efavirenz.

10. A compound for use according to claim 3, wherein the therapy directed to treating the retroviral infection comprises administering at least one protease inhibitor, wherein the at least one protease inhibitor is preferably selected from the group consisting of ritonavir, darunavir, atazanavir and combinations thereof.

11. A compound for use according to claim 3, wherein the therapy directed to treating the retroviral infection comprises administering at least one integrase inhibitor, wherein the at least one integrase inhibitor is preferably raltegravir.

12. A compound for use according to claim 3, wherein the therapy directed to treating the retroviral infection comprises administering one or more drug selected from the group consisting of a nucleoside reverse transcriptase inhibitor, a non-nucleoside reverse transcriptase inhibitor, a protease inhibitor, an integrase inhibitor, and combinations thereof.

13. A compound for use according to claim 3, wherein the therapy directed to treating the retroviral infection comprises administering two nucleoside reverse transcriptase inhibitors and one protease inhibitor, or wherein the therapy directed to treating the retroviral infection comprises administering two nucleoside reverse transcriptase inhibitors and one non-nucleoside reverse transcriptase inhibitor.

14. A compound for use according to claim 3, wherein the therapy directed to treating the retroviral infection comprises administering at least one drug selected from the group consisting of tenofovir, emtricitabine, efavirenz, raltegravir, ritonavir, darunavir, atazanavir, and combinations thereof.

## Patentansprüche

1. Verbindung, ausgewählt aus der Gruppe bestehend aus: 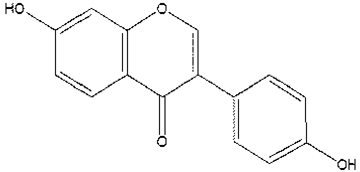 4',7-Dihydroxyisoflavon (Daidzein), 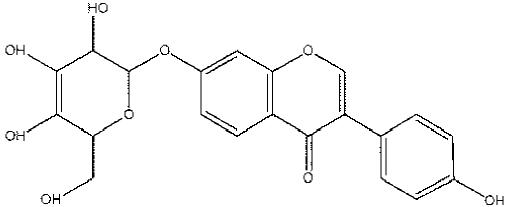 Daidzein-7-glucosid (Daidzin), 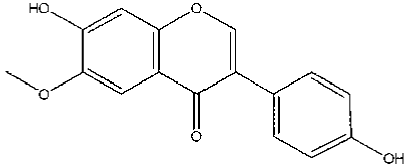 4',7-Dihydroxy-6-methoxyisoflavon (Glycitein), 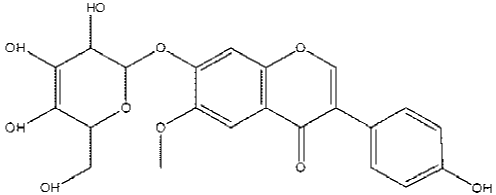 Glycitein-7-glucosid (Glycitin) oder Kombinationen davon zur Verwendung bei der Reaktivierung einer latenten retroviralen Infektion bei einem Patienten.

2. Verbindung, ausgewählt aus der Gruppe bestehend aus: 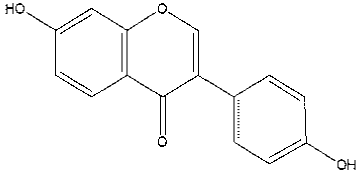 4',7-Dihydroxyisoflavon (Daidzein), 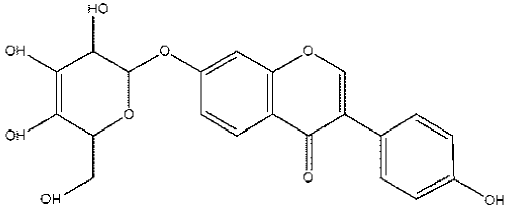 Daidzein-7-glucosid (Daidzin), 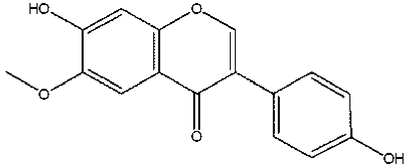 4',7-Dihydroxy-6-methoxyisoflavon (Glycitein), 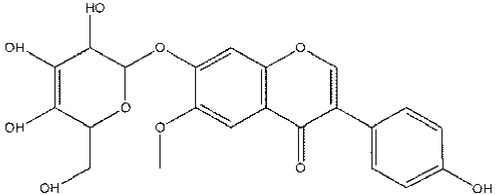 Glycitein-7-glucosid (Glycitin) oder Kombinationen davon,
zur Verwendung bei der Behandlung einer retroviralen Infektion bei einem Patienten, wobei die Behandlung ferner das Verabreichen einer Therapie umfasst, die darauf gerichtet ist, das Immunsystem des Patienten zu stimulieren.

3. Verbindung, ausgewählt aus der Gruppe bestehend aus: 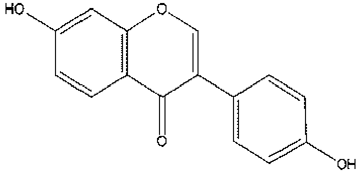 4',7-Dihydroxyisoflavon (Daidzein), 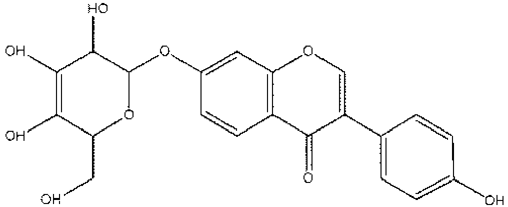 Daidzein-7-glucosid (Daidzin), 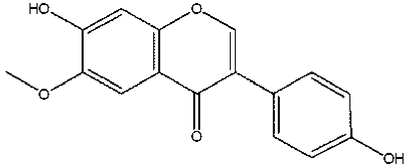 4',7-Dihydroxy-6-methoxyisoflavon (Glycitein), 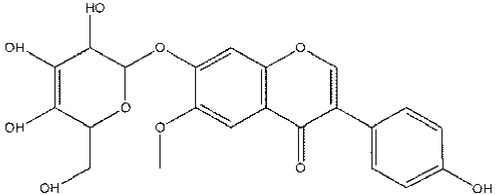 Glycitein-7-glucosid (Glycitin) oder Kombinationen davon zur Verwendung bei der Behandlung einer retroviralen Infektion bei einem Patienten, wobei die Behandlung ferner das Verabreichen einer Therapie umfasst, die darauf gerichtet ist, die retrovirale Infektion zu behandeln.

4. Verbindung zur Verwendung nach Anspruch 2, wobei die Therapie, die darauf gerichtet ist, das Immunsystem des Patienten zu stimulieren, dendritische zellbasierte Immuntherapie, T-Zell-Adoptivtransfer, autologe Immunverstärkungstherapie, gentechnisch veränderte T-Zellen, Immunwiederherstellung, Impfung oder Kombinationen davon umfasst.

5. Verbindung zur Verwendung nach Anspruch 2, wobei die Therapie, die darauf gerichtet ist, das Immunsystem des Patienten zu stimulieren, das Verabreichen eines oder mehrerer Zytokine an den Patienten beinhaltet.

6. Verbindung zur Verwendung nach Anspruch 5, wobei das eine oder die mehreren Zytokine ausgewählt sind aus der Gruppe bestehend aus Interleukin-2, Interleukin-4, Interleukin-5, Interleukin-7, Interleukin-10, transformierendem Wachstumsfaktor-a, Interferon-y und einer Kombination davon.

7. Verbindung zur Verwendung nach einem vorhergehenden Anspruch, wobei die latente retrovirale Infektion eine Infektion mit dem humanen Immundefizienzvirus (HIV) ist.

8. Verbindung zur Verwendung nach Anspruch 3, wobei die Therapie, die darauf gerichtet ist, die retrovirale Infektion zu behandeln, das Verabreichen von zumindest einem Nukleosid-Reverse-Transkriptase-Inhibitor umfasst, wobei der zumindest eine Nukleosid-Reverse-Transkriptase-Inhibitor vorzugsweise ausgewählt ist aus der Gruppe bestehend aus Tenofovir, Emtricitabin und Kombinationen davon.

9. Verbindung zur Verwendung nach Anspruch 3, wobei die Therapie, die darauf gerichtet ist, die retrovirale Infektion zu behandeln, das Verabreichen von zumindest einem Nicht-Nukleosid-Reverse-Transkriptase-Inhibitor umfasst, wobei der zumindest eine Nicht-Nukleosid-Reverse-Transkriptase-Inhibitor vorzugsweise Efavirenz ist.

10. Verbindung zur Verwendung nach Anspruch 3, wobei die Therapie, die darauf gerichtet ist, die retrovirale Infektion zu behandeln, das Verabreichen von zumindest einem Protease-Inhibitor umfasst, wobei der zumindest eine Protease-Inhibitor vorzugsweise ausgewählt ist aus der Gruppe bestehend aus Ritonavir, Darunavir, Atazanavir und Kombinationen davon.

11. Verbindung zur Verwendung nach Anspruch 3, wobei die Therapie, die darauf gerichtet ist, die retrovirale Infektion zu behandeln, das Verabreichen von zumindest einem Integrase-Inhibitor umfasst, wobei der zumindest eine Integrase-Inhibitor vorzugsweise Raltegravir ist.

12. Verbindung zur Verwendung nach Anspruch 3, wobei die Therapie, die darauf gerichtet ist, die retrovirale Infektion zu behandeln, das Verabreichen von einem oder mehreren Arzneimitteln umfasst, die aus der Gruppe ausgewählt sind, die aus einem Nukleosid-Reverse-Transkriptase-Inhibitor, einem Nicht-Nukleosid-Reverse-Transkriptase-Inhibitor, einem Protease-Inhibitor, einem Integrase-Inhibitor und Kombinationen davon besteht.

13. Verbindung zur Verwendung nach Anspruch 3, wobei die Therapie, die darauf gerichtet ist, die retrovirale Infektion zu behandeln, das Verabreichen von zwei Nukleosid-Reverse-Transkriptase-Inhibitoren und einem Protease-Inhibitor umfasst, oder wobei die Therapie, die darauf gerichtet ist, die retrovirale Infektion zu behandeln, das Verabreichen von zwei Nukleosid-Reverse-Transkriptase-Inhibitoren und einem Nicht-Nukleosid-Reverse-Transkriptase-Inhibitor umfasst.

14. Verbindung zur Verwendung nach Anspruch 3, wobei die Therapie, die darauf gerichtet ist, die retrovirale Infektion zu behandeln, das Verabreichen von zumindest einem Arzneimittel umfasst, das aus der Gruppe ausgewählt ist, die aus Tenofovir, Emtricitabin, Efavirenz, Raltegravir, Ritonavir, Darunavir, Atazanavir und Kombinationen davon besteht.

## Revendications

1. Composé choisi dans le groupe constitué par : 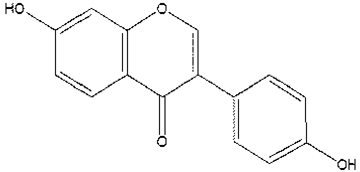 la 4',7-dihydroxyisoflavone (daidzéine), 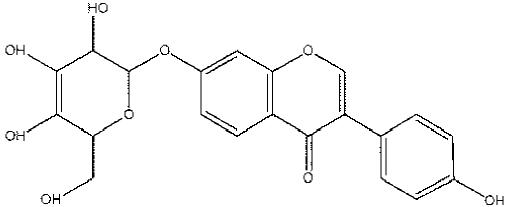 le 7-glucoside de daidzéine (daidzine), 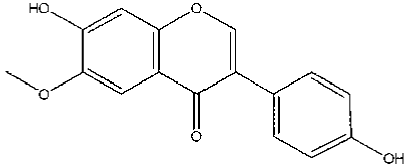 la 4',7-dihydroxy-6-méthoxyisoflavone (glycitéine), 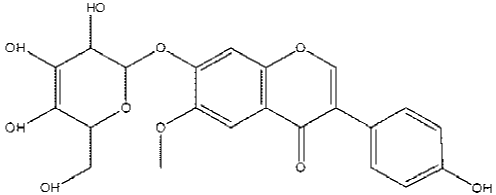 le 7-glucoside de glycitéine (glycitine) ou les combinaisons de ceux-ci, destinés à être utilisés dans la réactivation d'une infection rétrovirale latente chez un sujet.

2. Composé choisi dans le groupe constitué par : 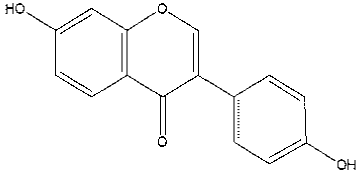 la 4',7-dihydroxyisoflavone (daidzéine), 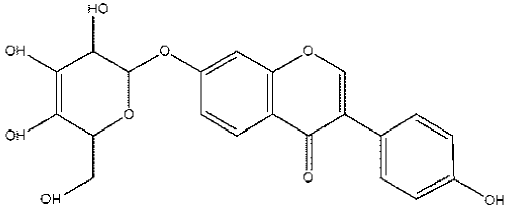 le 7-glucoside de daidzéine (daidzine), 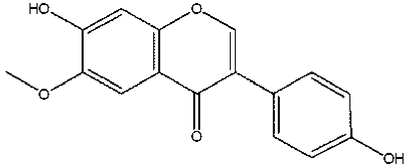 la 4',7-dihydroxy-6-méthoxyisoflavone (glycitéine), 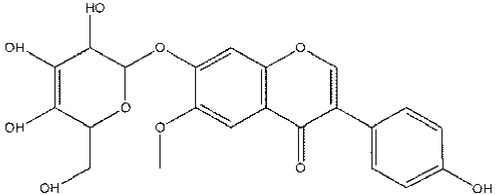 le 7-glucoside de glycitéine (glycitine) ou les combinaisons de ceux-ci,
destinés à être utilisés dans le traitement d'une infection rétrovirale chez un sujet, le traitement comprenant en outre l'administration d'une thérapie visant à stimuler le système immunitaire du sujet.

3. Composé choisi dans le groupe constitué par : 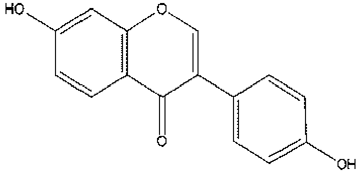 la 4',7-dihydroxyisoflavone (daidzéine), 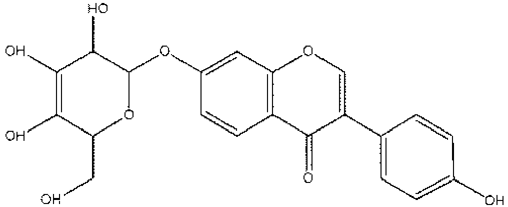 le 7-glucoside de daidzéine (daidzine), 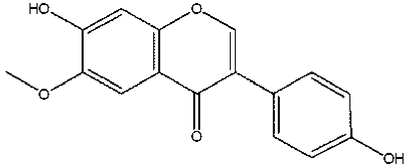 la 4',7-dihydroxy-6-méthoxyisoflavone (glycitéine), 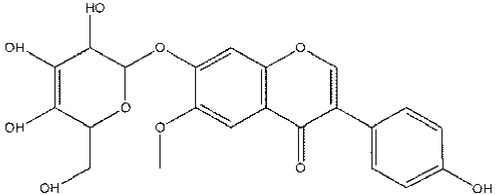 le 7-glucoside de glycitéine (glycitine) ou les combinaisons de ceux-ci, destinés à être utilisés dans le traitement d'une infection rétrovirale chez un sujet, le traitement comprenant en outre l'administration d'une thérapie visant à traiter l'infection rétrovirale.

4. Composé destiné à être utilisé selon la revendication 2, ladite thérapie visant à stimuler le système immunitaire du sujet comprenant une immunothérapie à base de cellules dendritiques, un transfert adoptif de lymphocytes T, une thérapie de renforcement immunitaire autologue, des lymphocytes T génétiquement modifiés, une récupération immunitaire, une vaccination, ou les combinaisons de ceux-ci.

5. Composé destiné à être utilisé selon la revendication 2, ladite thérapie visant à stimuler le système immunitaire du sujet comprenant l'administration d'une ou plusieurs cytokines au sujet.

6. Composé destiné à être utilisé selon la revendication 5, lesdites une ou plusieurs cytokines étant choisies dans le groupe constitué par l'interleukine-2, l'interleukine-4, l'interleukine-5, l'interleukine-7, l'interleukine-10, le facteur de croissance transformant-a, l'interféron-y et une combinaison de ceux-ci.

7. Composé destiné à être utilisé selon l'une quelconque des revendications précédentes, ladite infection rétrovirale latente étant une infection par le virus de l'immunodéficience humaine (VIH).

8. Composé destiné à être utilisé selon la revendication 3, ladite thérapie visant à traiter l'infection rétrovirale comprenant l'administration d'au moins un inhibiteur nucléosidique de la transcriptase inverse, ledit au moins un inhibiteur nucléosidique de la transcriptase inverse étant de préférence choisi dans le groupe constitué par le ténofovir, l'emtricitabine et les combinaisons de ceux-ci.

9. Composé destiné à être utilisé selon la revendication 3, ladite thérapie visant à traiter l'infection rétrovirale comprenant l'administration d'au moins un inhibiteur non nucléosidique de la transcriptase inverse, ledit au moins un inhibiteur non nucléosidique de la transcriptase inverse étant de préférence l'éfavirenz.

10. Composé destiné à être utilisé selon la revendication 3, ladite thérapie visant à traiter l'infection rétrovirale comprenant l'administration d'au moins un inhibiteur de la protéase, ledit au moins un inhibiteur de la protéase étant de préférence choisi dans le groupe constitué par le ritonavir, le darunavir, l'atazanavir et les combinaisons de ceux-ci.

11. Composé destiné à être utilisé selon la revendication 3, ladite thérapie visant à traiter l'infection rétrovirale comprenant l'administration d'au moins un inhibiteur de l'intégrase, ledit au moins un inhibiteur de l'intégrase étant de préférence le raltégravir.

12. Composé destiné à être utilisé selon la revendication 3, ladite thérapie visant à traiter l'infection rétrovirale comprenant l'administration d'un ou plusieurs médicaments choisis dans le groupe constitué par un inhibiteur nucléosidique de la transcriptase inverse, un inhibiteur non nucléosidique de la transcriptase inverse, un inhibiteur de la protéase, un inhibiteur de l'intégrase et les combinaisons de ceux-ci.

13. Composé destiné à être utilisé selon la revendication 3, ladite thérapie visant à traiter l'infection rétrovirale comprenant l'administration de deux inhibiteurs nucléosidiques de la transcriptase inverse et d'un inhibiteur de la protéase, ou ladite thérapie visant à traiter l'infection rétrovirale comprenant l'administration de deux inhibiteurs nucléosidiques de la transcriptase inverse et d'un inhibiteur non nucléosidique de la transcriptase inverse.

14. Composé destiné à être utilisé selon la revendication 3, ladite thérapie visant à traiter l'infection rétrovirale comprenant l'administration d'au moins un médicament choisi dans le groupe constitué par le ténofovir, l'emtricitabine, l'éfavirenz, le raltégravir, le ritonavir, le darunavir, l'atazanavir et les combinaisons de ceux-ci.
